# EUROPÄISCHE PATENTANMELDUNG

(11) **EP 1 574 158 A1**
(43) Veröffentlichungstag der Anmeldung: **14.09.2005**
(21) Anmeldenummer: 04024608.4
(22) Anmeldetag: 15.10.2004
(51) Int. Cl.: A47L 7/04, A47L 9/10, A47L 9/12, A47L 9/14, B01J 20/32, B01J 20/18, B01J 20/20, B01J 20/26, B01J 20/22

(54) **Adsorbens, Staubsammelraum sowie Verfahren zur Geruchsadsorption**

(30) Priorität: 01.03.2004 DE 102004009956
(71) Anmelder: Eurofilters N.V., 3900 Overpelt (BE)
(72) Erfinder: Schultink, Jan c/o Eurofilters N.V., 3900 Overpelt (BE); Sauer, Ralf, Dr. c/o Eurofilters N.V., 3900 Overpelt (BE)
(74) Vertreter: Pfenning, Meinig & Partner GbR

(57) **Zusammenfassung**

In Fig. 3 sind in der Zusammenstellung neben den Versuchen, die bereits in Fig. 2 enthalten sind und die als Versuchsreihe XII bezeichnet sind, noch Messergebnisse aus weiteren Versuchen enthalten.

Die Messergebnisse der Versuchsreihen X und XI beziehen sich dabei im Wesentlichen auf mit Aktivkohlen beschichtete Fasern. Wie aus Fig. 3 hervorgeht, zeichnet sich das erfindungsgemäße Adsorbens insbesondere dadurch aus, dass bereits bei geringsten Mengen an Adsorptionsmittel (z.B. 0,3 g Bambusknoten Aktivkohle) schon eine überdurchschnittlich hohe Minderung der Geruchsstoffkonzentration der Abluft erreicht wird.

## Beschreibung

Die Erfindung betrifft ein neues Adsorbens, insbesondere zur Geruchsabsorption. Das Adsorbens besteht aus einem spezifischen Trägermaterial und einem Adsorptionsmaterial. Die Erfindung betrifft weiterhin einen Staubsammelraum in dem das Adsorbens enthalten ist. Letztlich betrifft die Erfindung noch ein Verfahren zur Geruchsadsorption.

Im Stand der Technik sind bereits verschiedene Maßnahmen bekannt geworden, die zu einer Reduzierung von Gerüchen aus in Filtern abgeschiedenen Stäuben führen.

Eine Lösung besteht darin, dass die mit Geruchsstoffen belastete Luft durch ein separates nachgeschaltetes Filter geführt wird. Dabei kommen Schüttbettfilter aber auch Filter aus mit Kohle oder anderen Adsorbentien beschichteten Trägerstrukturen zum Einsatz. Eine derartige Lösung ist in der GB 2 288 749 beschrieben.

Im Stand der Technik ist es auch bekannt geworden durch Einbringen von mit Duftstoffen getränkten Körpern, in den Filterraum, eine Überdeckung von Gerüchen zu erreichen. Verwendet werden hierzu mit Parfüm getränkte Fasergebilde, die mit einer Kunststoffhülle ummantelt sind, Naturstoffe wie z. B. Orangenkerne oder Orangenschalen, Kunststoffteile die während des Spritzgussvorganges mit Parfüm oder natürlichen ätherischen Ölen beaufschlagt werden aber auch anorganische Trägermaterialien wie Sand/Carbonate, die mit Duftstoffen getränkt sind, (WO 94/21305). In der US 5,461,751 A werden mit antibakteriellen und/oder fungiziden Substanzen getränkte Granulate beschrieben.

Letztlich ist es auch der WO 01/08543 A1 bekannt, dass man ein Adsorbens in loser Form in einen Staubsammelfilter einbringt. Als Adsorbens werden dabei Aktivkohle, die in Bruch/Kugelform oder auch als Fasern vorliegen kann sowie Zeolithe und poröse Polymere angesprochen.

Die Lösung nach WO 01/08543 zeigt schon eine befriedigende Reduktion der Geruchsstoffe in der Ausblasluft von Handstaubsaugern. Allerdings hat diese Lösung auch einen gravierenden Nachteil. Bezüglich der gleichmäßigen Verteilung des Adsorbens in der Filtertüte ist es wünschenswert, ein möglichst feines, leichtes Adsorbens zu verwenden. Dies hat weiterhin den Vorteil, dass die innere Oberfläche des Adsorbens auf zahlreichen relativ kurzen Wegen (Zugangsporen) verfügbar ist. Bei der hohen Feinstaubbelastung in einer Staubsaugerfiltertüte, durch die die Zugangsporen leicht verstopft werden können, kann nur so gewährleistet werden, dass die innere Oberfläche näherungsweise vollständig zur Geruchsadsorption genutzt wird.

Sehr feine Partikel bleiben allerdings nicht wie gewünscht, homogen mit dem Staub gemischt innerhalb der Filtertüte, sondern dringen durch die innersten Filterlagen und werden größtenteils in der Filtertütenwand abgeschieden. Dadurch erhöht sich unerwünschterweise der Widerstand der Filtertüte (Druckverlustanstieg) und das Adsorbens steht nicht mehr als Geruchsbinder zur Verfügung. Durch die Verwendung relativ grobkörniger Adsorbentien lässt sich dieser Druckverlustanstieg der Filtertüte vermeiden. Die angestrebte gleichmäßige Verteilung des Adsorbens in der Filtertüte wird durch das höhere Gewicht der Teilchen allerdings verschlechtert. Zusätzlich werden die Zugangsporen schnell verstopft und nur noch ein kleiner Bruchteil der inneren Oberfläche steht zur Geruchsadsorption zur Verfügung.

Ausgehend von der WO 01/08543 A1 ist es die Aufgabe der vorliegenden Erfindung, ein neuartiges Adsorbens sowie einen Staubsammelraum anzugeben, mit dem eine gegenüber dem Stand der Technik verbesserte Reduzierung der Gerüche von in Staubsammelräumen abgeschiedenen Stäuben erreicht wird und bei dem das Adsorptionsmaterial effektiv eingesetzt wird.

Die Aufgabe wird in Bezug auf das Adsorbens durch die Merkmale des Patentanspruches 1, betreffend den Staubsammelraum durch die Merkmale des Patentanspruches 35 gelöst. Die Unteransprüche geben vorteilhafte Weiterbildungen an.

Gemäß der vorliegenden Erfindung wird somit vorgeschlagen, dass das Adsorbens aus Fasern, Flocken und/oder Granulat als Trägermaterial besteht, auf die ein pulverförmiges Adsorptionsmaterial aufgebracht ist. Die Anmelderin konnte zeigen, dass dann, wenn ein derartiges Adsorbens z. B. in einem Staubsammelraum verwendet wird, gegenüber dem Stand der Technik nur ein Bruchteil des Adsorptionsmaterials eingesetzt werden muss. Mit dem erfindungsgemäßen Adsorbens wird dabei gleichzeitig eine deutliche Reduzierung von Gerüchen von den in Staubsammelräumen abgeschiedenen Stäuben erreicht. Dies ist offensichtlich darauf zurückzuführen, dass das in Fasern und/oder Flocken vorliegende Adsorbens unter den Betriebsbedingungen, z.B. eines Staubsammelfilters im Staubsammelfilter aufgewirbelt vorliegt und sich damit homogen mit dem Staub mischt.

Der Einsatz von reinem pulverförmigen Adsorptionsmaterial mit einer vergleichbaren mittleren Korngröße wie es auf den Fasern/Flocken aufgebracht ist, ist normalerweise nicht möglich, da sonst das Filtermaterial leicht verstopft wird.

Bei dem Adsorbens hat es sich als vorteilhaft erwiesen, wenn das Adsorptionsmaterial in einer Menge von 1 bis 40 Gew.-%, bevorzugt in einer Menge von 7 bis 25 Gew.-% des Trägermaterials aufgebracht ist. Das Adsorptionsmaterial in Pulverform kann dabei ganzflächig oder auch nur bereichsweise auf der Oberfläche des Trägermaterials aufgebracht sein.

Aus stofflicher Sicht sind beim Adsorptionsmaterial an und für sich alle aus dem Stand der Technik bekannten pulverförmigen Materialien einsetzbar. Besonders geeignet sind dabei Aktivkohle auf Basis von Stein-, Holz-, Bambus- oder Kokosnussschalenkohle, sauer oder basisch oder mit Silbersalzen imprägnierte Aktivkohle, funktionalisierter Kohlenstoff, hydrophobe Zeolithe und/oder hydrophobe, poröse Polymere.

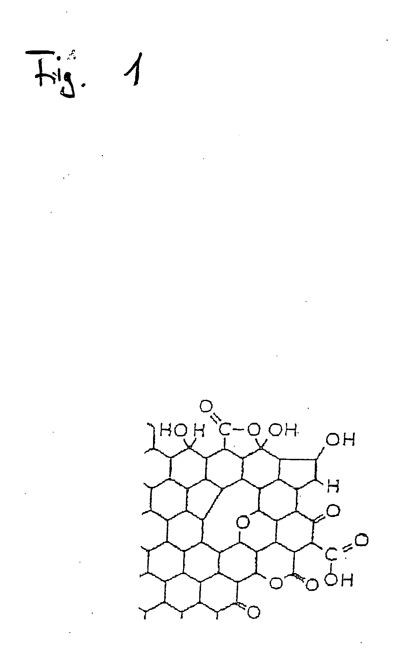

Die Anmelderin konnte zeigen, dass neben pulverförmiger Aktivkohle auch funktionalisierter Kohlenstoff in Form eines aromatischen Kohlenstoffgerüstes mit funktionellen Gruppen besonders geeignet ist. Ein derartiges Adsorbens ist unter der Bezeichnung Carbonized Basal Plates (CBP) bekannt geworden. Eine Beschreibung dieser Materialien ist bei R. Kunz, 1816 North Cascade Avenue, Colorado Springs zu finden. In Fig. 1 ist die Struktur eines derartigen Adsorbens wiedergegeben. Dieses Adsorbens hat sich als besonders geeignet erwiesen. Als weiterhin geeignet hat sich pulverisierte Bambusknoten Aktivkohle erwiesen. Ein derartiges Adsorbensmaterial ist z.B. von Aqua Air Adsorbens in DE-04509 Krostitz unter der Bezeichnung BW 200 erhältlich.

Bevorzugt ist es, wenn die Aktivkohle in einer mittleren Korngröße von 1 bis 100, bevorzugt 15 bis 50 µm eingesetzt wird.

Es hat sich gezeigt, dass neben der vorstehend beschriebenen Aktivkohle ausgewählte Zeolithe besonders geeignet sind. Wesentlich für die Eignung ist zunächst, dass die Mikroporen des Zeolithen eine ausreichende Größe haben. Erst oberhalb eines Durchmessers von 5 Å sind die Mikroporen in der Lage typische Geruchsmoleküle aufzunehmen und zu binden. Zusätzlich muss der Zeolith einen stark hydrophoben (unpolaren) Charakter haben. Erst ab einem Verhältnis von SiO₂/Al₂O₃ > 200 (Modul) ist ein Zeolith ausreichend unpolar um die Geruchsmoleküle zu binden. Besonders stallinen metallorganischen Komplexen wie z.B. "MOF-177". Dieses Adsorbens realisiert eine extrem große Oberfläche (4500 m²/g) bei ausreichend großen Mikroporen (10 Å). Diese Kristalle sind beschrieben in Nature Bd. 427, Seiten 523 bis 527, Februar 2004. Auf dem Offenbarungsgehalt dieses Dokumentes wird Bezug genommen.

Als Trägermaterial für das erfindungsgemäße Adsorbens werden Fasern, Flocken und/oder Granulat vorgeschlagen.

Aus stofflicher Sicht können für das Trägermaterial des Adsorbens in Bezug auf die Fasern Chemiefasern und/oder Naturfasern eingesetzt werden. Bei den Chemiefasern wären cellulosische Fasern wie Viskose oder synthetische Fasern zu nennen. Beispiele für synthetische Fasern sind Fasern aus Polyolefinen, Polyester, Polyamiden, Polyacrylmethyl und/oder Polyvinylalkohol.

Beispiele für Naturfasern sind Cellulose, Holzfaserstoffe, Kapok, Flachs, Jute, Manilahanf, Kokos, Wolle, Baumwolle, Kenaf, Abaca, Maulbeerbast und/oder Fluffpulp.

Es hat sich weiterhin gezeigt, dass es bei den Fasern bevorzugt ist, wenn diese verzweigt, gekrimt, hohl und oder texturiert sind und/oder einen nicht kreisförmigen (z. B. trilobalen) Querschnitt aufweisen.

Von den Abmessungen her ist es günstig, wenn die Fasern eine mittlere Länge zwischen 0,3 mm und 100 mm, bevorzugt zwischen 0,5 und 70 mm aufweisen.

Die synthetischen Fasern können auch antibakteriell ausgerüstet sein. Dies kann dadurch erfolgen, dass bereits bei der Herstellung antibakterielle Stoffe zugesetzt werden. Der Vorteil dieser Fasern besteht darin, dass die antibakteriellen Stoffe praktisch nicht freigesetzt werden und keine Minderung der antibakteriellen Wirkung eintritt. Solche Fasern sind erhältlich bei Rhovyl in F-55310 Tronville en Barrois, z.B. die Fasern Rhovyl'A.S.+® oder bei Japan Exlan Co. Ltd., Tokyo sowie bei Sterling Fibers Inc., 5005 Sterling Way, Pace, Fla unter dem Markennamen "biofresh" und DAK Americas, 5925 Carnegie Blvd., Charlotte, NC 28209.

Selbstverständlich ist es auch möglich, die Fasern nachträglich antibakteriell auszurüsten.

Gemäß der vorliegenden Erfindung ist es weiterhin vorgesehen, dass als Trägermaterial nicht nur Fasern eingesetzt werden, sondern auch Flocken. Als geeignete Materialien wären hierbei zu nennen Schaumstoffe, Vliesstoffe, Textilien, geschäumte Stärke, geschäumte Polyolefine sowie Folien.

Bei den Flocken sind Durchmesser von 0,3 bis 30 mm, bevorzugt 0,5 bis 20 mm vorteilhaft. Besonders günstig ist ein Durchmesser von 1 bis 9,5 mm.

Erfindungsgemäß kann als Trägermaterial auch ein Granulat eingesetzt werden. Das Granulat kann stofflich aus den gleichen Verbindungen wie die Fasern ausgewählt sein. Die Herstellung erfolgt nach üblichen Methoden. Die Größe des Granulats (Durchmesser) liegt im Bereich von 1 bis 9,5 mm.

Das erfindungsgemäße Adsorbens ist dabei so aufgebaut, dass auf dem Trägermaterial wie vorstehend beschrieben, das pulverförmige Adsorbtionsmaterial chemisch und/oder physikalische auf die Oberfläche des Trägermaterials aufgebracht ist.

Ein Aufbringen im Sinne der vorliegende Erfindung kann so erfolgen, dass erwärmtes Adsorptionsmaterial auf die Oberflächen des Trägermaterials aufgebracht wird, so dass durch Wärmeübertragung auf die Oberfläche des Trägermaterials ein Anschmelzen erfolgt und die pulverförmigen Partikel haften. Andererseits kann auch die Oberfläche des Trägermaterials erweicht werden und dann die Partikel auf deren Oberfläche aufgebracht werden. Bei Bikomponentenfasern ist es möglich, dass die äußere Schicht einen niedrigeren Schmelzpunkt als der Kern aufweist, so dass durch dessen Erwärmung eine Haftung der Partikel möglich ist.

Physikalisch kann das Aufbringen dadurch erfolgen, dass elektrostatisch geladene Fasern eingesetzt werden. Es kann mit triboelektrisch oder mit durch Coronaladung aufgeladenen Fasern gearbeitet werden. Bevorzugt werden insbesondere auch geladene Splitfasern. Es ist auch möglich, die Haftung des Adsorptionsmaterials an der Faser dadurch zu erreichen, dass geeignete Fasern und Adsorptionsmittel gemischt werden, wobei durch den triboelektrischen Effekt die Fasern und die Adsorbtionsteilchen entgegengesetzt aufgeladen werden. Man erreicht so eine hervorragende elektrostatische Bindung des Adsorbens an die Fasern, ohne die Oberfläche der Adsorbensteilchen durch Bindemittel zu reduzieren. Besonders vorteilhaft lässt sich diese Möglichkeit bei der Variante synthetische Fasern mit makroporösen Polymeren einsetzen. So zeigt beispielsweise die Kombination Polypropylen-Fasern mit SDVB Pulver beim Mischen eine starke triboelektrische Aufladung.

Das Adsorbens wie vorstehend beschrieben, kann auch in einer luftdurchlässigen Umhüllung vorliegen. Der Vorteil dieser Ausführungsform ist darin zu sehen, dass das Adsorbens leicht handhabbar ist und dann, wenn es z.B. in einem Staubsammelfilter in einem Staubsauger eingesetzt wird, problemlos in den Staubsammelfilter eingebracht werden kann. Die Umhüllung für einen derartigen Anwendungsfall ist dabei so aufgebaut, dass sie unter den Betriebsbedingungen wieder zerstört wird, so dass das Adsorbens im Staubsammelfilter aufgewirbelt und in Zirkulation gehalten werden kann. Geeignete Materialien sind hierfür Vliese, z. B. Nonwoven mit geringer Grammatur z. B. Meltblown mit 5 gr/m².

Die Erfindung betrifft weiterhin einen Staubsammelraum, (Patentansprüche 35 bis 50). Der Staubsammelraum nach der Erfindung zeichnet sich nun dadurch aus, dass ein Adsorbens wie vorstehend beschrieben, enthalten ist. Es hat sich als günstig herausgestellt, wenn im Staubsammelraum pro 1000 cm³ Volumen 0,2 bis 5 g des Adsorbens enthalten sind. Besonders bevorzugt liegt die Menge des Adsorbens pro 1000 cm³ bei 0,3 bis 2 g Adsorbens. Bei einem Staubsammelraum wie vorstehend beschrieben, handelt es sich bevorzugt um sog. beutellose Staubsauger, wie z.B. Zyklonstaubsauger.

Für Zyklonstaubsauger ist es günstig, wenn poröse Polymere als Adsorbtionsmaterialien eingesetzt werden, da dies keine zusätzliche Verschmutzung durch Kohleabrieb oder Unterkornanteil verursacht und ein Verkratzen der meist transparenten Sammelbehälter vermieden wird. Gemäß der vorliegenden Erfindung wird unter einem Staubsammelraum auch ein Abfallsammelbehälter verstanden.

Gemäß der vorliegenden Erfindung werden unter einem Staubsammelraum insbesondere auch solche verstanden, bei denen der Staubsammelraum durch einen Staubsammelfilter gebildet wird, der aus einem von Luft durchströmbaren Filtermaterial besteht. Um eine optimale Wirkung des Adsorbens im Staubsammelfilter zu erreichen, ist es bevorzugt, wenn dieses von vorne herein in loser Form in den Staubsammelfilter eingefüllt wird oder aber, dass das Adsorbens in einem in einer luftdurchlässigen Umhüllung aufweisenden Beutel im Staubsammelfilter vorliegt. Der Beutel kann dann an einer Stelle z. B. direkt im Auftreffbereich der Strömung fixiert sein. Das Adsorbens kann auch in loser Form in einem Teilbereich der Tüteninnenfläche liegen und von einer dünnen luftdurchlässigen Vliesschicht abgedeckt sein (Tasche). Dieser Bereich kann auch als durchgehender Streifen ausgebildet sein. Das Adsorbens kann auch in einem Kissen vorliegen. Hierbei wird ein Kissen eingesetzt, das aus mindestens einer Lage eines Filterpapiers oder eines speziellen Vliesstoffes besteht, auf dem das Adsorbens liegt. Das Adsorbens ist dabei dann mit mindestens einer Lage eines Vliesstoffes abgedeckt. Dieser Vliesstoff ist dabei so ausgelegt, dass unter den Betriebsbedingungen eine Zerstörung des Vliesstoffes eintritt. Selbstverständlich muss das Kissen so angeordnet sein, dass das Filterpapier/der spezielle Vliesstoff direkt auf der Innenseite der Filtertüte angebracht ist und das leichte Vlies direkt von der Luftströmung getroffen wird. Eine besonders bevorzugte Ausführungsform der luftdurchlässigen Umhüllung ist ein Kissen, das gebildet wird aus einer Lage Filterpapier mit einer Luftdurchlässigkeit > 250 1/m²/s einer Füllung mit dem erfindungsgemäßen Adsorbens und einer Lage eines Vliesstoffes mit einem Flächengewicht < 10 g/m². Dieses Kissen wird z.B. durch punktuelles Verkleben so im Staubsammelraum fixiert, dass die Papierlage des Kissens dem Filtermaterial des Staubsammelraums zugewandt ist. In diesem Zusammenhang wird der Offenbarungsgehalt der WO 2004/052500 A1, EP 1 426 090 A1 und EP 1 415 699 B1 genannt.

Derartige Staubsammelfilter sind bevorzugt Staubsaugerbeutel. Diese sind dabei üblicherweise so dimensioniert und ausgelegt, dass sie mit einem Volumenstrom von 10 m³/Std. bis 400 m³/Std. durchströmbar sind. Es ist dabei bevorzugt, wenn im Staubsammelfilter pro 1000 cm³ Volumen 0,2 bis 5 g des Adsorbens enthalten sind, besonders bevorzugt 0,3 bis 2 g Adsorbens. Bei kleineren Mengen wurde festgestellt, dass dann keine ausreichende geruchsreduzierende Wirkung erreicht wird und bei größeren Mengen ist nachteilig, dass dann der Staubsammelraum als solches schon mit einem zu großen Volumen an Adsorbens gefüllt ist.

Der Staubsammelfilter nach der Erfindung besteht aus stofflicher Sicht dabei bevorzugt aus einem Filtermaterial, das ein ein- oder mehrschichtiges Papier und/oder Vliesmaterial sein kann. Derartige Filtermaterialien sind z. B. für Staubsaugerbeutel bekannt. Hierzu wird auf die EP-A 0 960 645 A1 verwiesen. Bei dem Staubsammelfilter nach der Erfindung kann es sich z. B. um einen Staubsaugerbeutel oder aber auch um einen plissierten Filter oder einen Taschenfilter handeln.

Letztlich betrifft die Erfindung ein Verfahren zur Geruchsadsorption in einen Staubsammelraum (Patentansprüche 51 bis 56).

Das erfindungsgemäße Verfahren zur Geruchsadsorption. zeichnet sich dadurch aus, dass ein Adsorbens wie vorstehend beschrieben, eingesetzt wird. Bevorzugt wird dabei im Staubsammelraum mit 0,2 bis 5 g Adsorbens pro 1000 cm³ gearbeitet.

Beim erfindungsgemäßen Verfahren wird als Staubsammelraum bevorzugt ein Staubsammelfilter aus von Luft durchströmbarem Filtermaterial eingesetzt. Beim Verfahren ist dabei wichtig, dass das Adsorbens während des Betriebes des Staubsammelfilters lose im Staubsammelfilter vorliegt. Bevorzugt ist dabei der Staubsammelfilter ein Staubsaugerbeutel. Das Adsorbens wird somit entweder bei der Herstellung oder kurz nach der Herstellung in den Staubsammelfilter eingebracht und so ausgeliefert. Beim erstmaligen Gebrauch unter einem gegebenen Volumenstrom kommt es dann zu einer Aufwirbelung des Adsorbens im abgeschlossenen Staubsammelfilter und das Adsorbens kann seine wie vorstehend beschriebene geruchsmindernde Wirkung entfalten. Selbstverständlich ist es auch möglich, dass bei Beginn des Saugvorgangs das Adsorbens eingebracht wird, nämlich in der Weise, dass das Adsorbens aufgesaugt wird.

Das Adsorbens kann weiterhin in einer Umhüllung vorliegen und wieder wie vorstehend beschrieben, bereits von vorneherein im Staubsaugerbeutel enthalten sein oder aber das Adsorbens wird mit der Umhüllung vor Beginn des Saugvorgangs in den Staubsaugerbeutel eingebracht oder es wird direkt aufgesaugt.

Beim erfindungsgemäßen Verfahren ist es besonders günstig, dass auch nachträglich, d. h. direkt mit Beginn des Saugvorgangs noch das Adsorbens eingebracht werden kann, da dadurch auch bereits alle bisher gängigen Filtertüten in ihrer geruchsmindernden Wirkung einfach durch Aufsaugen oder Einbringen des Adsorbens vor dem erstmaligen Saugvorgang verbessert werden können. Selbstverständlich kann, wenn erforderlich, auch eine Nachdosierung erfolgen. Besonders bevorzugt handelt es sich beim erfindungsgemäßen Verfahren um ein Verfahren zum Staubsaugen mit einem Bodenstaubsauger oder einem Handstaubsauger und der Staubsammelfilter ist ein Staubsaugerbeutel.

Die Erfindung wird nachfolgend anhand eines Beispieles und der Fign. 1 bis 3 näher erläutert.
- Fig. 1: zeigt den Aufbau von funktionalisiertem Kohlenstoff wie er bevorzugt als Adsorbtionsmittel eingesetzt wird.
- Fig. 2: zeigt graphisch den Verlauf der Geruchsstoffkonzentration der Abluft bei verschiedenen Proben.
- Fig. 3: zeigt eine tabellarische Zusammenstellung

### Beispiel:

Im Folgenden werden anhand von Beispielen die durchgeführten Versuche beschrieben.

### 1) Messvorbereitung:

Die Bodenstaubsauger, Typ Miele S512-1 wurden vor der Messreihe mehrere Stunden lang mit einer leeren Filtertüte betrieben, um einen evtl. vorhandenen Geruch des Aggregats zu minimieren. Einen Tag vor dem eigentlichen Messbeginn wurde in jedes der Aggregate eine Filtertüte eingesetzt. Danach wurde jedes Aggregat komplett abgeklebt, um so ein Abblasen der Abluft durch eine andere Öffnung als die gebohrte Probennahmeöffnung (Durchmesser 13 mm) zu verhindern. Danach wurden die Aggregate in den auf 20° C eingestellten Wärmeschrank eingestellt. Weiterhin wurde die für die gesamte Messreihe nötige Menge Kaffee aus einem 500 g Vakuumpack entnommen, abgewogen und in 5 g Portionen eingeschweißt.

### 2. Versuchsablauf:

Die Versuchsfläche zum Aufsaugen des Kaffee/Staubgemisches besteht aus einer Platte Laminat mit der Grundfläche 1,21 m x 1,85 m, entsprechend 2,24 m². Die Bodenstaubsauger wurden mit der Bürsteneinstellung "Teppich" betrieben. Zur Untersuchung der Geruchsminderung im Staubsaugerbeutel wurden für jedes Aggregat an den Untersuchungstagen 1 bis 6 50 g Teststaub Typ 8 (DMT, Zusammensetzung: 70 % Mineralstaub, 20 % Arbocell, 10 % Linters) sowie 5 g Kaffee (10 % Kaffee in Bezug auf die Staubmenge) gleichmäßig auf der Versuchsfläche verteilt. Am 7. und 8. Versuchstag wurden je 100 g Staub und 10 g Kaffee verteilt, am letzten Versuchstag wurde nur noch beprobt. Nach dem Verteilen des Kaffee/Staubgemisches wurde der Saugfuß auf ein sauberes Stück der Versuchsfläche aufgesetzt und bei einer geringen Saugleistung (300 Watt) wurde ein Probenbeutel mit einer Gesamtlänge von 1,5 m (Füllmenge ca. 15 Liter) direkt aus der Probenahmeöffnung der abgeklebten Bodenstaubsauger befüllt. Nach dem Abnehmen der Probenbeutel (bei vollständiger Füllung) und Ausschalten des Aggregates wurde die Abklebung komplett entfernt und die Saugleistung auf maximale Leistung (1.600 Watt) hochgeregelt. Dann wurde über eine Zeitdauer von zwei Minuten das Kaffee/Staubgemisch von der Versuchsfläche abgesaugt. Nach der Probennahme wurden die Aggregate abgeschaltet, wieder komplett abgeklebt und bis zur nächsten Beprobung im Wärmeschrank gelagert. Es wurde an jedem Versuchstag Temperatur und Luftfeuchte bei der Entnahme aus dem Wärmeschrank sowie bei der Probennahme erfasst.

### 3) Untersuchte Varianten und Ausstattung der Aggregate

Alle Aggregate wurden mit Motorschutzfilter betrieben.
Variante A
   Aggregat A, 3 g Splitfaser/MN 200 MR 4636 lose in der Filtertüte,
Variante B
   Aggregat B, 3 g Splitfaser/DALY lose in der Filtertüte,
Variante C
   Aggregat C, 3 g Splitfaser/MN 200 MR 4638 lose in der Filtertüte,
Variante D
   Aggregat D, Nullvariante, leere Filtertüte,
Variante E
   Aggregat E, 3 g Splitfaser/TZB 2014 lose in der Filtertüte,
Variante F
   Aggregat F, 3 g Splitfaser/DAY lose in der Filtertüte,

### 4) Mess- und Analysenverfahren

### 4.1 Geruchsemissionen

### 4.1.1 Messverfahren; Grundlagen des Verfahrens

Bestimmung der Geruchsstoffkonzentration gemäß europäischer Norm DIN EN 13725.

### 4.1.2 Probennahmematerial

Die Probenluft wird bei der statischen Probennahme in einen Folienbeutel gezogen. Als Probenbeutel werden handelsübliche Folienschläuche verwendet, die aus geruchsfreiem Material (Nalophan NA©) bestehen, welches einerseits nahezu gasdicht ist und andererseits praktisch keine Geruchsstoffe adsorbiert.

### 4.1.3 Olfaktometer

Die Olfaktometrie stellt eine kontrollierte Darbietung von mit Geruchsstoffen beladener Luft sowie eine Erfassung der dadurch beim Menschen auftretenden Sinnesempfindungen dar. Mit dem Olfaktometer wird eine Gasprobe (Geruchsstoffprobe) mit Neutralluft verdünnt und Testpersonen (Probanden) als Riechprobe dargeboten. Ein Probandenkollektiv besteht aus vier Riechern sowie einem Versuchsleiter, der für die Bedienung des Olfaktometers während eines Messvorgangs zuständig ist.

Für die beschriebenen Messungen wurde ein rechnergesteuertes Olfaktometer T09 mit vier Probandenplätzen und automatischer Auswertung verwendet. Die Messungen wurden entsprechend DIN EN 13725 durchgeführt. Das Olfaktometer wurde mit Druckluft über eine Filtergruppe mit Silicagel (Entfeuchtung), Aktivkohle (Geruchsstoffabscheidung), Wattefilter und Glasfaser-Mikrofeinfilter (Staubabscheidung) betrieben. Die Messungen wurden entsprechend den DIN EN 13725 nach der Ja-Nein-Methode durchgeführt.

Geruchsstoffmengen werden in Geruchseinheiten (GE) gemessen, wobei eine GE der Stoffmenge eines Geruchsstoffes oder eines Stoffgemisches entspricht, die - bei 20° C und 1013 hPa in 1 m³ Neutralluft verteilt - entsprechend der Definition der Geruchsschwelle bei 50 % eines Probandenkollektivs eine Geruchswahrnehmung auslöst. Die Geruchsstoffkonzentration an der Geruchsschwelle beträgt definitionsgemäß 1 GE/m³.

Analog zum Schall werden Geruchsstoffpegel bezüglich der Schwellenkonzentration von 1 GE/m³ definiert. Dabei entspricht z.B. eine Geruchsstoffkonzentration von 100 GE/m³ einem Geruchsstoffpegel von 20 dB.

### 4.1.4 Beschreibung des Probandenkollektivs

Gemessen wurde bei den olfaktometrischen Messungen mit Versuchsleiter und vier Probanden entsprechend DIN EN 13725.

### 4.1.5 Auswertung der Proben

Die olfaktometrische Messung der Proben erfolgte maximal vier Stunden nach der Probenahme.

### 4.1.6 Anzahl der Messreihen je Messtag

12 Geruchsstoffkonzentrationsmessungen, mit je drei Reihen pro Messung. Je zwei Geruchsstoffkonzentrationsmessungen mit n-Butanol.

### 4.1.7 Weitere Untersuchungen

Um die Ergebnisse zusätzlich abzusichern, wurden die Proben an allen Messtagen auf Intensität und Hedonik (Direktbeurteilung aus dem Probenbeutel in Anlehnung an die VDI Richtlinie 3882) untersucht.

Dabei wurden vom für die Versuchsreihe typischen Kaffee(Staubgeruch) abweichende Gerüche durch die Probanden charakterisiert.

Bei den Versuchsreichen wurden Raumtemperatur und - feuchte während der Probenahme an den Messtagen erfasst sowie die Temperatur und Feuchte im jeweiligen Wärmeschrank bei der Entnahme der Aggregate.

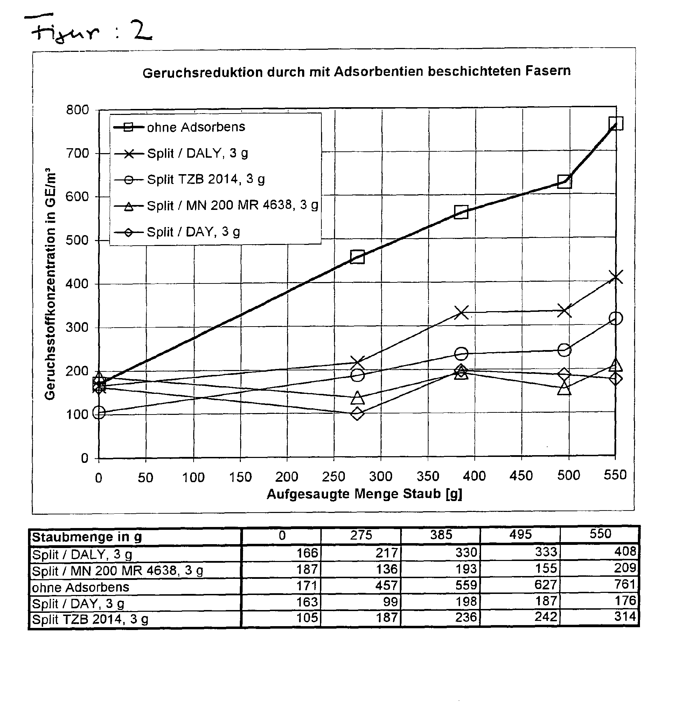

Fig. 2 zeigt den Verlauf der Geruchsstoffkonzentration der Abluft von Staubsaugern der 5 untersuchten Varianten der beschichteten Fasern im Vergleich zu einer Nullvariante. Fig. 2 macht deutlich, dass alle untersuchten Varianten eine deutliche Reduzierung der Geruchstoffkonzentration der Abluft bewirken. Überraschend ist hierbei insbesondere die gute Wirkung bei der sehr geringen Menge an Adsorbtionsmittel. Schon mit 0,3 g Adsorbtionsmittel ist eine deutliche Reduktion der Geruchsstoffkonzentration erzielbar. Die heute üblichen Lösungen verwenden dagegen 10 g Aktivkohle.

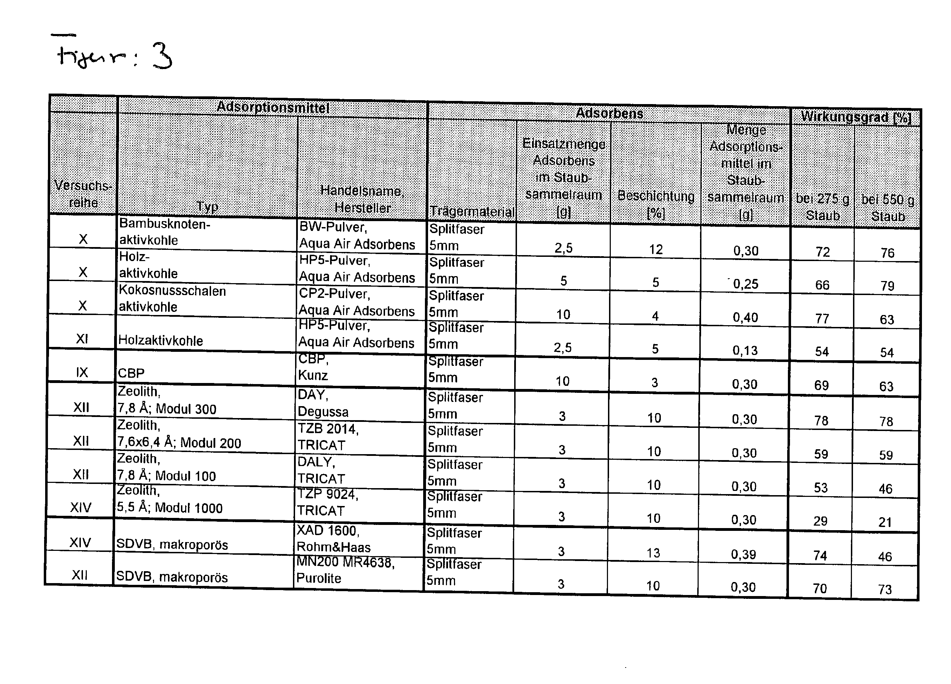

Fig. 3 zeigt die Ergebnisse in einer tabellarischen

## Patentansprüche

1. Adsorbens für Staubsammelfilter, insbesondere zur Geruchsadsorption, **dadurch gekennzeichnet, dass** das Adsorbens aus Fasern, Flocken und/oder Granulat als Trägermaterial besteht, auf die oberflächlich ein pulverförmiges Adsorptionsmaterial aufgebracht ist.

2. Adsorbens nach Anspruch 1, **dadurch gekennzeichnet,**
**dass** das Adsorptionsmaterial in einer Menge von 1 bis 50 Gew.-% des Trägermaterials aufgebracht ist.

3. Adsorbens nach Anspruch 2, **dadurch gekennzeichnet,**
**dass** 7 bis 25 Gew.-% aufgebracht sind.

4. Adsorbens nach mindestens einem der Ansprüche 1 bis 3, **dadurch gekennzeichnet,**
**dass** das Adsorptionsmaterial ausgewählt ist aus Aktivkohle, imprägnierter Aktivkohle, funktionalisiertem Kohlenstoff, hydrophobe Zeolithe, hydrophobe, poröse Polymere und/oder kristalline metallorganische Komplexe.

5. Adsorbens nach Anspruch 4, **dadurch gekennzeichnet,**
**dass** es sich bei dem funktionalisierten Kohlenstoff um ein aromatisches Kohlenstoffgerüst mit funktionellen Gruppen handelt.

6. Adsorbens nach Anspruch 4, **dadurch gekennzeichnet, dass** es sich bei der Aktivkohle um Kokosnussschalen-, Holz-, Stein- oder Bambuskohle handelt.

7. Adsorbens nach Anspruch 4 oder 5, **dadurch gekennzeichnet, dass** die Aktivkohle mit sauren oder basischen Chemikalien und/oder mit Silbersalzen imprägniert ist.

8. Adsorbens nach Anspruch 4, **dadurch gekennzeichnet, dass** die Zeolithe Mikroporen mit einer Porengröße > 5 Å besitzen.

9. Adsorbens nach Anspruch 8, **dadurch gekennzeichnet, dass** die Porengröße der Mikroporen > 6,5 Å ist.

10. Adsorbens nach Anspruch 8 oder 9, **dadurch gekennzeichnet, dass** die spezifische Oberfläche der Zeolithe > 400 m²/g ist.

11. Adsorbens nach mindestens einem der Ansprüche 8 bis 10, **dadurch gekennzeichnet, dass** die Zeolithe ein Modul > 200, bevorzugt > 300 aufweisen.

12. Adsorbens nach mindestens einem der Ansprüche 8 bis 11, **dadurch gekennzeichnet, dass** die Partikelgröße der Zeolithe im Bereich von 2 bis 30 µm liegt.

13. Adsorbens nach Anspruch 4, **dadurch gekennzeichnet, dass** die porösen Polymere Mikroporen von 6 bis 20 Å, Mesoporen von 20 bis 500 Å und Makroporen > 500 Å aufweisen.

14. Adsorbens nach Anspruch 13, **dadurch gekennzeichnet, dass** der durchschnittliche Porendurchmesser zwischen 3 und 300 Å liegt.

15. Adsorbens nach Anspruch 13 oder 14, **dadurch gekennzeichnet, dass** die Partikelgröße im Bereich von 1 bis 500 µm, bevorzugt 1 bis 200 µm liegt.

16. Adsorbens nach mindestens einem der Ansprüche 13 bis 15, **dadurch gekennzeichnet, dass** das Porenvolumen > 0,8 cm³/g ist.

17. Adsorbens nach mindestens einem der Ansprüche 13 bis 15, **dadurch gekennzeichnet, dass** die porösen Polymere hydrophob sind.

18. Adsorbens nach mindestens einem der Ansprüche 13 bis 17, **dadurch gekennzeichnet, dass** die porösen Polymere aufgebaut sind aus Polystyrol und/oder deren Derivaten.

19. Adsorbens nach mindestens einem der Ansprüche 1 bis 18, **dadurch gekennzeichnet,**
**dass** das Adsorptionsmaterial chemisch und/oder physikalisch an das Trägermaterial gebunden ist.

20. Adsorbens nach mindestens einem der Ansprüche 1 bis 19, **dadurch gekennzeichnet,**
**dass** das Adsorptionsmaterial an ein elektrostatisch geladenes Trägermaterial gebunden ist.

21. Adsorbens nach mindestens einem der Ansprüche 1 bis 20, **dadurch gekennzeichnet,**
**dass** das Adsorptionsmaterial pulverförmig ist und eine mittlere Korngröße von 1 bis 100 µm aufweist.

22. Adsorbens nach mindestens einem der Ansprüche 1 bis 21, **dadurch gekennzeichnet,**
**dass** die Fasern Chemiefasern und/oder Naturfasern sind.

23. Adsorbens nach Anspruch 22, **dadurch gekennzeichnet,**
**dass** die Fasern antibakteriell ausgerüstet sind.

24. Adsorbens nach Anspruch 22 oder 23, **dadurch gekennzeichnet,**
**dass** die Chemiefasern cellulosische Fasern, wie Viskose und/oder synthetische Fasern sind.

25. Adsorbens nach Anspruch 24, **dadurch gekennzeichnet,**
**dass** die synthetischen Fasern ausgewählt sind aus Fasern aus Polyolefinen, Polyester, Polyamiden, Polyacrylnitril und/oder Polyvinylalkohol.

26. Adsorbens nach Anspruch 22 oder 23, **dadurch gekennzeichnet,**
**dass** die Naturfasern ausgewählt sind aus Cellulose, Holzfaserstoffe, Kapok, Flachs, Jute, Manilahanf, Kokos, Wolle, Baumwolle, Kenaf, Abaca, Maulbeerbast und/oder Fluffpulp.

27. Adsorbens nach mindestens einem der Ansprüche 1 bis 26, **dadurch gekennzeichnet,**
**dass** die Fasern glatt, verzweigt, gekrimpt, hohl und/oder texturiert sind und/oder einen nicht kreisförmigen (z. B. trilobalen) Querschnitt aufweisen.

28. Adsorbens nach mindestens einem der Ansprüche 1 bis 27, **dadurch gekennzeichnet,**
**dass** die Fasern eine mittlere Länge zwischen 0,3 mm und 100 mm, bevorzugt zwischen 0,5 und 70 mm aufweisen.

29. Adsorbens nach Anspruch 28, **dadurch gekennzeichnet,**
**dass** die Fasern eine mittlere Länge von 1 bis 9,5 mm aufweisen.

30. Adsorbens nach mindestens einem der Ansprüche 1 bis 29, **dadurch gekennzeichnet,**
**dass** die Flocken ausgewählt sind aus Schaumstoffen, Vliesstoffen, Textilien, geschäumter Stärke, geschäumte Polyolefine, sowie Folien und Reißfasern.

31. Adsorbens nach Anspruch 30, **dadurch gekennzeichnet,**
**dass** die Flocken einen Durchmesser von 0,3 mm bis 30 mm, bevorzugt 0,5 bis 20 mm, aufweisen.

32. Adsorbens nach Anspruch 31, **dadurch gekennzeichnet,**
**dass** die Flocken einen Durchmesser von 1 bis 9,5 mm aufweisen.

33. Adsorbens nach mindestens einem der Ansprüche 1 bis 32, **dadurch gekennzeichnet,**
**dass** das Adsorbens in einer luftdurchlässigen Umhüllung eingeschlossen ist.

34. Adsorbens nach Anspruch 33, **dadurch gekennzeichnet,**
**dass** die Umhüllung ein luftdurchlässiges Vlies ist.

35. Staubsammelraum, insbesondere für einen Staubsauger, der mit Luft beaufschlagbar ist,
**dadurch gekennzeichnet ,**
**dass** im Staubsammelraum ein Adsorbens nach einem der Ansprüche 1 bis 34, enthalten ist.

36. Staubsammelraum nach Anspruch 35, **dadurch gekennzeichnet,**
**dass** im Staubsammelraum pro 1000 cm³ Volumen 0,2 bis 5 g des Adsorbens enthalten sind.

37. Staubsammelraum nach Anspruch 36, **dadurch gekennzeichnet,**
**dass** pro 1000 cm³ 0,3 bis 2 g Adsorbens enthalten sind.

38. Staubsammelraum nach mindestens einem der Ansprüche 35 bis 37, **dadurch gekennzeichnet, dass** er der Staubsammelraum eines beutellosen Staubsaugers ist.

39. Staubsammelraum nach mindestens einem der Ansprüche 35 bis 37, **dadurch gekennzeichnet, dass** er durch einen Abfallsammelbehälter gebildet ist.

40. Staubsammelraum nach mindestens einem der Ansprüche 35 bis 37, **dadurch gekennzeichnet,**
**dass** er durch einen Staubsammelfilter aus einem von Luft durchströmbaren Filtermaterial gebildet ist.

41. Staubsammelraum nach Anspruch 40, **dadurch gekennzeichnet,**
**dass** das Adsorbens in einem in einer luftdurchlässigen Umhüllung aufweisenden Beutel im Staubsammelfilter vorliegt.

42. Staubsammelraum nach Anspruch 40 oder 41, **dadurch gekennzeichnet,**
**dass** das Adsorbens in einem Teilbereich der Innenfläche des Staubsammelfilters unter einer Abdeckung angeordnet ist.

43. Staubsammelraum nach Anspruch 42, **dadurch gekennzeichnet,**
**dass** die Abdeckung eine Vliesschicht ist.

44. Staubsammelraum nach Anspruch 42, **dadurch gekennzeichnet,**
**dass** das Adsorbens in einem Kissen enthalten ist, das auf einem Teilbereich der Innenfläche des Staubsammelfilters angeordnet ist.

45. Staubsammelraum nach Anspruch 44, **dadurch gekennzeichnet,**
**dass** das Kissen aus mindestens einer Schicht eines Filterpapiers oder eines speziellen Vlieses besteht, wobei das auf der Oberfläche des Filterpapier angeordnete Adsorbens durch mindestens eine Vliesschicht abgedeckt ist.

46. Staubsammelraum nach mindestens einem der Ansprüche 41 bis 45, **dadurch gekennzeichnet,**
**dass** das Umhüllungsmaterial des Beutels bzw. die Abdeckung aus einem unter Betriebsbedingungen zerstörbaren Material gebildet ist.

47. Staubsammelraum nach mindestens einem der Ansprüche 40 bis 46, **dadurch gekennzeichnet,**
**dass** der Staubsammelfilter so dimensioniert und ausgelegt ist, dass er mit einem Volumenstrom von 10 cm³/h bis 400 m³/h betrieben werden kann.

48. Staubsammelraum nach mindestens einem der Ansprüche 40 oder 47, **dadurch gekennzeichnet,**
**dass** das Filtermaterial des Staubsammelfilters ein ein- oder mehrschichtiges Papier und/oder Vliesmaterial ist.

49. Staubsammelraum nach mindestens einem der Ansprüche 40 bis 48, **dadurch gekennzeichnet,**
**dass** er durch einen Staubsaugerbeutel gebildet ist.

50. Staubsammelraum nach mindestens einem der Patentansprüche 40 bis 48, **dadurch gekennzeichnet,**
**dass** er durch ein plissierter Filter oder Taschenfilter gebildet ist.

51. Verfahren zur Geruchsadsorbtion in einem Staubsammelraum nach mindestens einem der Ansprüche 40 bis 50, **dadurch gekennzeichnet,**
**dass** mit einem Adsorbens nach einem der Ansprüche 1 bis 34 gearbeitet wird.

52. Verfahren nach Anspruch 51, **dadurch gekennzeichnet,**
**dass** 0,2 bis 5 g Adsorbens pro 1000 cm³ Staubsammelraum verwendet wird.

53. Verfahren nach Anspruch 51 oder 52, **dadurch gekennzeichnet, dass** als Staubsammelraum ein von Luft durchströmbarer Staubsammelfilter eingesetzt wird.

54. Verfahren nach Anspruch 53, **dadurch gekennzeichnet,**
**dass** vor Beginn eines erstmaligen Saugvorgangs oder bei Beginn des Saugvorgangs das Adsorbens in den Staubsammelfilter eingebracht wird.

55. Verfahren nach mindestens einem der Ansprüche 53 oder 54, **dadurch gekennzeichnet,**
**dass** das Adsorbens in einer Umhüllung vorliegt und vor Beginn eines erstmaligen Saugvorgangs oder bei Beginn des Saugvorgangs in den Staubsammelfilter eingebracht wird.

56. Verfahren nach Anspruch 55, **dadurch gekennzeichnet,**
**dass** die Umhüllung so ausgebildet ist, dass sie unter dem gegebenen Volumenstrom zerstört wird.

57. Verfahren nach mindestens einem der Ansprüche 53 bis 56 **dadurch gekennzeichnet,**
**dass** es sich um ein Verfahren zum Staubsaugen mit einem Bodenstaubsauger oder einem Handstaubsauger handelt.

58. Verwendung des Adsorbens nach mindestens einem der Ansprüche 1 bis 34 zur Geruchsadsorption.
